# EUROPEAN PATENT SPECIFICATION

(11) **EP 2 252 236 B1**
(45) Date of publication and mention of the grant of the patent: **04.12.2013**
(21) Application number: 09701339.5
(22) Date of filing: 05.01.2009
(51) Int. Cl.: A61F 2/01

(54) **VEIN FILTER**
VENENFILTER
FILTRE VEINEUX

(30) Priority: 11.01.2008 US 10837
(43) Date of publication of application: 24.11.2010
(73) Proprietor: Rex Medical, L.P., Conshohocken, PA 19428 (US)
(72) Inventor: MCGUCKIN, James, F., Radnor, Pennsylvania 19087 (US); BRESSLER, James, E., Langhorne, PA 19047 (US); SCHALLER, David, M., Wallingford, PA 19086 (US)
(74) Representative: Jackson, Derek Charles
(86) International application number: PCT/US2009/000019
(87) International publication number: WO 2009/088970

(56) References cited:
- EP-A- 1 894 543
- WO-A-2006/036457
- US-A- 4 425 908
- US-A1- 2005 027 314
- US-A1- 2007 173 885
- US-A1- 2007 213 685

## Description

### BACKGROUND

### Technical Field

This application relates to a vascular filter and more particularly to a vein filter for capturing blood clots within the vessel.

### Background of Related Art

Passage of blood clots to the lungs is known as pulmonary embolism. These clots typically originate in the veins of the lower limbs and can migrate through the vascular system to the lungs where they can obstruct blood flow and therefore interfere with oxygenation of the blood. Pulmonary embolisms can also cause shock and even death.

In some instances, blood thinning medication, e.g. anticoagulants such as Heparin, or sodium warfarin can be given to the patient. These medications, however, have limited use since they may not be able to be administered to patients after surgery or stroke or given to patients with high risk of internal bleeding. Also, this medication approach is not always effective in preventing recurring blood clots.

Therefore, surgical methods to reduce the likelihood of such pulmonary embolisms by actually blocking the blood clot from reaching the lungs have been developed. To this end, minimally invasive surgical techniques have been developed involving the placement of a mechanical barrier in the inferior vena cava. These barriers are in the form of filters and are typically inserted through either the femoral vein in the patient's leg or the right jugular vein in the patient's neck or arm under local anesthesia. The filters are then advanced intravascularly to the inferior vena cava where they are expanded to block migration of the blood clots from the lower portion of the body to the heart and lungs.

These prior filters take various forms. One type of filter is composed of coiled wires such as disclosed in U.S. Patent Nos. 5,893,869 and 6,059,825. Another type of filter consists of legs with free ends having anchors for embedding in the vessel wall to hold the filter. These filters are disclosed, for example, in U.S. Patent Nos. 4,688,553, 4,781,173, 4,832,055, and 5,059,205, 5,984,947 and 6,007,558. Another type of filter is disclosed in U.S. Patent no. 6,214,025 consisting of wires twisted together to form a cylindrical anchoring portion conforming to the inner vessel wall surface to exert a radial force and a conical filtering portion.

Several factors have to be considered in designing vein filters. One factor is that the filter needs to be securely anchored within the vessel wall, while avoiding traumatic engagement and damage to the wall as well as damage to the neighboring abdominal aorta. Another factor is that the filter must be collapsible to a sufficiently small size to be easily maneuvered and atraumatically advanced intravascularly to the inferior vena cava or other target vessel. Thirdly, the filter should direct the blood clots to the center of the vessel to improve dissolution of the clot within the vessel by the blood flow.

The filters disclosed in the commonly assigned co-pending application 10/889,429 (hereinafter "the '429 application") (US-A-2005165442) satisfy the foregoing parameters. The filters have sufficient anchoring force to retain the filter within the vessel while providing atraumatic contact with the vessel wall, have a minimized insertion (collapsed) profile to facilitate delivery through the vascular system to the surgical site, and direct migration of the captured blood clots to the center of the vessel. The filters also provide simplified insertion through the femoral or the right jugular vein or arm into the inferior vena cava.

The filters of the '429 application can advantageously be readily removed minimally invasively, e.g. intravascularly, from the patient, thus advantageously providing for a temporary filter. Thus, these filters advantageously strike the balance of having structure to provide sufficient anchoring while enabling atraumatic removal from the vessel after a period of time. Certain filters of the '429 application also advantageously have a retrieval end configured to facilitate grasping by a snare as well as to facilitate withdrawal by providing a smooth transition into a retrieval sheath.

The filters of the '429 are very effective in achieving their desired functions, whether used as a permanent or temporary filter. The filter of commonly assigned U.S. application serial no. 11/888,929, filed August 3, 2007, (US-A-2008221609) discloses a modification to the filters of the '429 patent to further facilitate removal if used as a temporary filter. This is achieved by providing spacers at an end of the filter.

WO-A-2006036457 describes a vessel filter having a first region having a first set of struts forming a mounting portion and a filter portion having a converging region at a first portion to direct particles toward the centre of the filter. The mounting portion is flared in the expanded position to have a transverse dimension increasing toward a second portion opposite the first portion. A second set of struts forms a second mounting portion flared in the expanded position. A plurality of spacer struts extend between the first and second regions. The second set of struts may form a second filter portion.

US-A-2007173885 describes a compact retrievable blood clot filter which has a filter section, a releasable lock and an alignment section connected to the filter section. Alignment ribs of the alignment section have releasable upstream ends that are locked to the filter by the releasable lock. The releasable upstream ends of the alignment ribs are capable of being released from the releasable lock so that during retrieval of the filter, the alignment ribs can slide through the endothelial tissue that may have grown around the alignment ribs.

US-A-2007213685 describes a method of removing an implanted vessel filter by a femoral approach comprising the steps of providing a catheter with a curved tip, inserting a straightening device into the catheter to move the catheter tip from a curved position to a more straightened position, advancing the catheter tip through the femoral vein and past a cranial end of the filter, withdrawing the straightening device to enable the catheter tip to return to the curved condition, and inserting a filter grasping device through the catheter and the curved catheter tip to exit a distal portion to grasp the filter.

US-A-4425908 describes a blood clot filter that is inwardly radially collapsible into a collapsed configuration for inserting into a vein, and upon insertion automatically radially expands into a predetermined functional form which is in contact with the inner wall of the vein. In the expanded configuration, the filter comprises a plurality of wires in the form of overlapping loops, with portions of the loops contacting the inner wall of the vein, providing a filter basket at the leading end of the filter; at the trailing end the wires have circumferentially spaced leg portions whose free ends contact the inner wall of the vein. The filter wires are composed of a material having a first, relatively pliable low-temperature condition and a second, relatively rigid high-temperature condition. A guide wire feeder device introduces the filter in its collapsed configuration into the blood vessel of a patient through a standard angiographic catheter. Upon emerging from the catheter, the filter responds to the ambient temperature and assumes its functional form.

US-A-2005027314 describes blood clot filters which have self-centering capabilities when placed in a blood vessel. A blood clot device includes a number of filter legs formed at least in part of shape-memory material configured to transform from a centering configuration to a filtering configuration when deployed in the body. An attachment section on a distal section of each filter leg is configured to pierce and secure the filter to the vessel wall at a first location. A bend region may be heat set into the shape-memory material to provide a second contact location along the vessel wall to aid in centering the filter within the vessel. The bend region can be formed by heating the shape-memory material above its final austenite temperature, and then shaping the filter leg to form a pad that abuts the vessel wall during deployment.

It would be advantageous to provide a filter with improved spacers to keep the retrieval end spaced from the vessel wall to thereby further facilitate removal of the filter.

### SUMMARY

The present invention provides a vessel filter comprising a first region, a second region and a third region, the filter movable between a collapsed position for delivery to the vessel and an expanded position for placement within the vessel, the first region having a filter portion having a converging region to direct particles toward the center of the filter, the first region including a plurality of spaced apart elongated struts, and a plurality of connecting struts extending at an angle from the elongated struts to form closed geometric shapes, the second region being flared in the expanded position having a transverse dimension increasing toward a second end portion opposite the filter portion, the second end portion being at the proximal region of the filter, the second region including vessel engaging hooks at the second end portion, the third region having a plurality of spacer struts extending radially with respect to a longitudinal axis of the filter in the expanded position, wherein the spacer struts form a plurality of integral closed wing-shaped looped struts positioned distally of the filter portion, each loop extending a distance from a central longitudinal axis of the filter that is less than a distance the vessel engaging hook is from the central longitudinal axis.

In the preferred embodiment, a transverse dimension of the filter at the region of the looped spacer struts is less than a transverse dimension of the filter at the region of the vessel engaging portions.

The spacer struts may be substantially aligned with the longitudinal axis of the filter in their collapsed position. In the expanded configuration an opening in the loops may be transverse to a longitudinal axis of the filter.

In a preferred embodiment, the filter is formed from a laser cut tube and composed of shape memory material and the spacer struts are formed integrally with the filter.

In a preferred embodiment, the converging region terminates in a tubular portion and each of the elongated struts in the first region extends outwardly from the tubular portion, and the spacer struts extend radially from the tubular portion and are positioned distally of the elongated struts (with respect to the direction of blood flow). The spacer struts preferably have a looped shape memory position and during delivery have a collapsed position substantially flush with the tubular portion.

Spacer struts may extend radially from a first end of the filter in a first direction toward the proximal region of the filter to an end region and then curve inwardly in a second opposite direction toward the distal end of the filter.

The filter may include a first tubular portion proximal of the spacer struts and a second tubular portion distal of the spacer struts, the first and second tubular portions dimensioned to receive a guidewire therethrough.

The spacer struts may be self expanding upon exposure from a delivery sheath.

The filter may further comprise a retrieval hook at a distal end of the filter, the hook laterally offset from a central longitudinal axis of the filter to facilitate passage of a guidewire.

The filter may comprise a body made from a single tube, the tube cut to create the plurality of elongated struts and spacer struts formed integrally with the filter.

The filter may include interconnecting struts in the filtering region of the body to form closed geometric shapes.

A wall of the filter may separate the looped struts from the filter portion.

A plurality of elongated struts may extend integrally from a tubular portion and curve outwardly therefrom, each of the struts terminating in integrally formed vessel engaging hooks at a proximal end portion, an intermediate portion of the tube forming the filter portion, and the plurality of closed looped struts are positioned distally of the filter portion and formed integrally with the elongated struts, each loop extending a distance from a central longitudinal axis of the filter that is less than a distance the vessel engaging hook is from the central longitudinal axis.

### BRIEF DESCRIPTION OF THE DRAWINGS

Preferred embodiment(s) of the present disclosure are described herein with reference to the drawings wherein:
Figure 1 is a perspective view of a vein filter not according to the present invention in a collapsed (retracted) configuration, and shown removed from a delivery tube/sheath;
Figure 2 is a perspective view of the vein filter of Figure 1 in an expanded (radially extending) configuration;
Figure 2A is an enlarged view of the hooks of the filter of Figure 2; and
Figure 3 is a perspective view of an embodiment of the vein filter of the present invention shown in an expanded (radially extending) configuration.

### DETAILED DESCRIPTION OF PREFERRED EMBODIMENTS

Turning now to the drawings, wherein like reference numerals identify similar or like components throughout the several views, the vein filters are described for placement within the inferior vena cava to capture blood clots or other particles which could otherwise pass to the lungs.

The filter is movable from a low profile collapsed configuration to facilitate insertion through the delivery sheath to a larger expanded placement configuration to enable atraumatic engagement with the vessel walls to secure (mount) the filter within the inferior vena cava. The filter is preferably substantially bell-shaped and preferably has a flared or mounting region (portion/section) and a filtering region (portion/section). The filtering region has inwardly directed struts, terminating in a converging region, thereby directing particles toward the central axis of the filter. By directing the particles to the center, they will be exposed to greater blood flow (since there is greater flow at the center than near the wall of the vessel) which improves dissolution of the particles. The filter increases in transverse dimension to form a flared region. The flare provides less contact area than a straight region, resulting in less tissue ingrowth to facilitate removal of the filter if desired. The flare also reduces the chance of vessel distortion if inserted into a curved vena cava. The filter also has a third region having spacers positioned distally (with respect to the direction of blood flow) of the filtering region to space the cranial end of the filter from the vessel wall to facilitate removal.

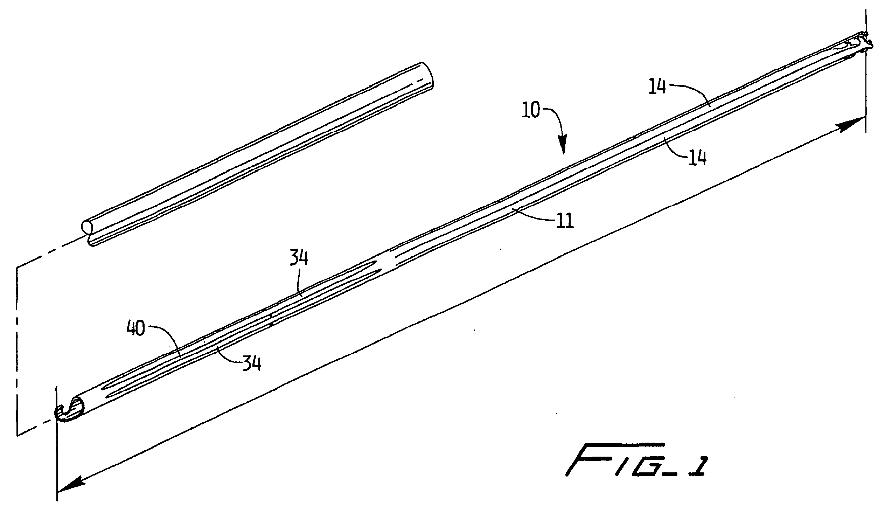

Turning now to details of the filter and with initial reference to Figure 1, the filter is designated generally by reference numeral 10 and is shown in a collapsed configuration for delivery. Filter 10 is preferably formed from a single tube 11 such that the struts and portions of the filter are integrally formed. In a preferred embodiment, the filter tube 11 is composed of shape memory material, such as Nitinol, a nickel titanium alloy, or elgiloy, however, other materials such as stainless steel are also contemplated. A plurality of cutouts are formed in the filter 10, preferably by laser cutting although other techniques are contemplated. In the illustrated embodiment, six elongated cutouts are formed, creating six strips or struts 14 of substantially uniform width separated by the cutouts. A second plurality of cutouts 34 are formed, forming the spacer struts described in detail below.

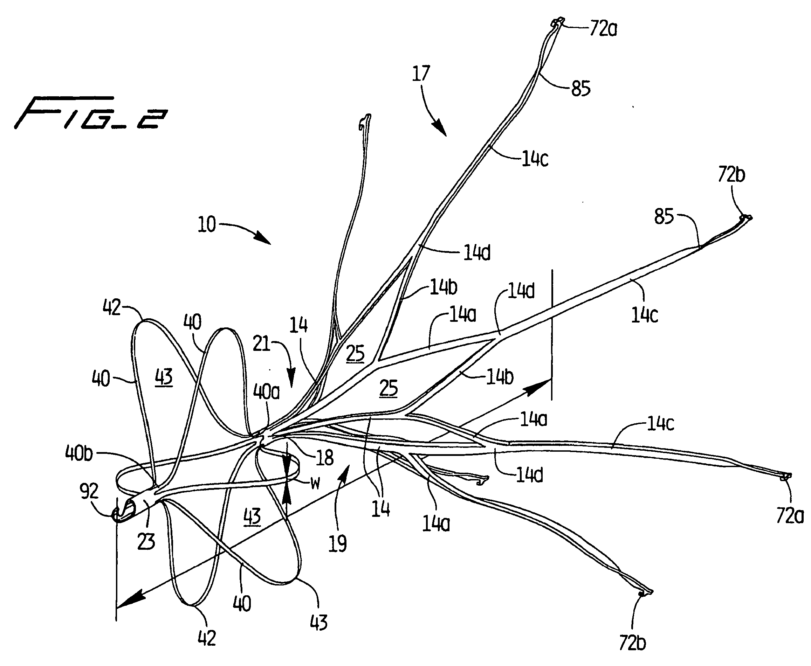

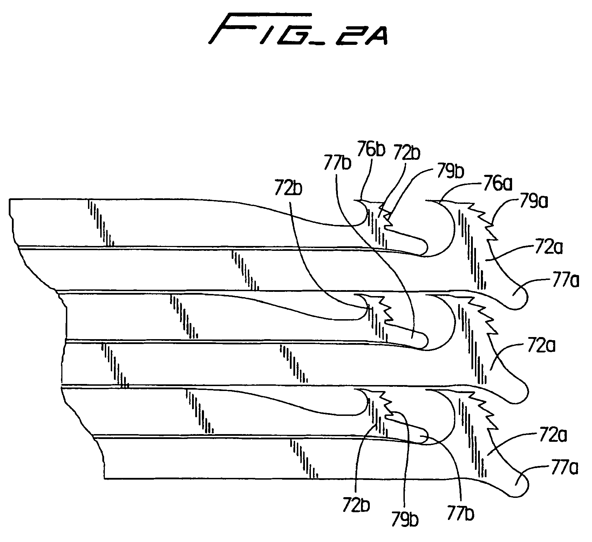

The collapsed configuration of filter 10 reduces the overall profile to facilitate delivery to the site. The diameter or transverse dimension of filter 10 in the collapsed configuration is preferably about 2mm and more preferably about 1.7mm. Other dimensions are also contemplated. The filter is thus preferably dimensioned for insertion through a 6 French delivery system and through a 6 French catheter. The diameter or transverse dimensions of the filter in the expanded placement configurations (e.g. Fig. 2 is greater than the diameter or transverse dimension in the collapsed (delivery) configuration of Figure 1.

Figure 2 illustrates the expanded placement configuration of the filter 10. Filter 10 is generally bell-shaped in configuration. Filter 10 has a flared region 17 and a converging region 21 at the filtering section 19. The transverse dimension of the filter at the flared (or mounting/anchoring) region 17 is greater than the transverse dimension at filtering section 19. Diameters (or transverse dimensions) preferably range from about 18mm to about 32mm, depending on the internal diameter of the vessel wall as will be explained in more detail below. Other dimensions are also contemplated. The elongated struts 14 are spaced apart as shown and extend at an angle away from the longitudinal axis L of filter 10 in region 17 to provide a flare. Preferably, this angle or taper is about 8°, although other dimensions are contemplated. When expanded, the six struts 14, as shown, are preferably spaced approximately 60 degrees apart. It is also contemplated that a fewer or greater number of struts and spacing other than 60 degrees could be provided.

Filtering section 19 extends from the flared region 17, and extends toward the central longitudinal axis of the filter 10 and converges into tubular portion 18.

The struts 14 of filter 10 terminate in hooks 72a, 72b which extend substantially perpendicular from the strut, achieved by torquing the struts at the region 85 so the hooks bend out of the plane. A first set of hooks 72a is larger than a second set of hooks 72b (see Figure 2A). Preferably when formed in a laser cut tube, hooks 72a are formed so that they occupy a region equivalent to the transverse dimension of two adjacent struts. Smaller hooks 72b are spaced axially with respect to each other and axially inwardly with respect to larger hooks 72a as in the filter hooks of the '429 application to minimize the collapsed profile (transverse dimension) of the filter when collapsed for insertion. The penetrating tips 76a, 76b of hooks 72a, 72b, respectively, penetrate the tissue to retain the filter, preferably temporarily, and point distally, toward the cranial (or distal with respect to blood flow) end of the filter.

Each of the hooks 72a, 72b preferably has a series of teeth 79a, 79b, and a heel 77a, 77b extends past (proximally or caudal of) the respective hook 72a, 72b to function as a stop to prevent the filter strut portions from going through the vessel wall. For clarity, not all of the hooks are fully labeled. This hook configuration is described in detail in commonly assigned patent application serial no. 11/888,929, filed August 3, 2007, (hereinafter referred to as the '929 application) (US-A-2008221609).

The six filter struts or strut portions 14 curve outwardly from tubular portion 18, extend integrally and radially therefrom and divide into two connecting filter struts or strut portions 14a, 14b (preferably of equal width, although differing dimensions are contemplated) that angle way from each other (in different directions) to extend to the connecting strut portion of an adjacent strut 14. Thus, connecting strut portion 14a of one strut 14 interconnects with the connecting strut portion 14b of an adjacent strut at joining region 14d. This forms closed geometric shapes 25, preferably substantially diamond shaped in configuration. For clarity, not all of the identical parts are labeled in the drawing.

In the illustrated example, preferably six struts are provided forming twelve interconnecting struts, however a different number of struts and closed geometric shapes can be provided. Note that although all six struts 14 are shown interconnected, it is also contemplated that fewer than all the struts can be interconnected. Also, the strut width can vary as described with respect to the filters disclosed in the '429 application.

After convergence of strut portions 14a, 14b at joining region 14d, it transitions into elongated integrally extending mounting strut portions 14c which form flared mounting or anchoring region 17. The length of the strut portions 14c in the anchoring region 17 can vary, with increased/decreased length increasing the flexibility/rigidity of the struts. The thickness of the strut portions can also vary to affect flexibility/rigidity.

As in the other examples described in the '429 application, terms such as interconnected, joined, etc., are used for ease of description, it being understood that preferably these portions are integral as they are preferably formed from a single tube. Also, mounting struts and filter struts used to describe the various embodiments disclosed herein can be considered as mounting strut "portions" or "sections" and filter strut "portions" or "sections" of the same struts if the filter is formed integrally, e.g. from a cut tube.

The tubular portion 23 is spaced distally (with respect to the direction of blood flow) of tubular portion 18 and is at the cranial end of filter 10. Tubular portion 23 preferably terminates in retrieval hook 92 as described with respect to the embodiment of Figure 20 in the '429 application. Other retrieval structure can also be utilized. Hook 92 is described briefly below.

A plurality of slots are formed in a third region of the tube during manufacture, preferably by laser cutting, to enable six strips to be formed creating spacers 40 for the filter at the cranial portion. In the collapsed position, spacers 40 are in a substantially aligned position with respect to tubular portions 18 and 23, i.e. substantially flush with the tubular portions. The tubular portions 18, 23 (and 118, 123 described below) are configured and dimensioned to receive a guidewire therethrough for inserting the filter over a guidewire. Spacers 40 are maintained in this collapsed position during delivery to the surgical site. (see e.g. Figure 1). The spacers 40 have a shape memorized position forming closed loops being substantially U-shaped shown in Figure 2 extending radially from the filter. Thus, once exposed from the delivery sheath, the spacers 40 self expand as they move from their collapsed position to their shape memory looped position of Figure 2. One or more of the curved surfaces 42 of the loops 40 can engage the vessel wall to maintain centering of the cranial end of the filter and to space tubular portion 23 and retrieval hook 92 away from the vessel wall. This spacing limits tissue ingrowth around the hook, thereby making it easier to grasp and remove filter 10.

The loops of spacers 40 are closed loops with end 40a extending from tubular wall 18 and end 40b extending from tubular wall 23. As shown, the looped struts 40 are positioned distally of the filter portion and proximally of the hook 92, with respect to the direction of blood flow and internal integrally from tubular portion 18. In the expanded, shape memorized position shown in Figure 2, the loop extends such that its apex, i.e. the region at the height of the loop, is generally in the middle portion of the loop 40. Thus, these substantially symmetrical spacer loops extend, when viewed from tubular wall 23, initially radially outwardly and then radially inwardly. An opening 43 through the loop 40 is transverse to the longitudinal axis of the filter.

Each loop 40 preferably has a thickened portion (not shown) at the region adjacent the tubular walls (portions) 18 and 23 to increase the strength at the transition. Also, the loops are preferably rectangular in cross-section, having a width W greater than its height. Although six loops are shown approximately 60 degrees apart, different distances apart are also contemplated. Also, a fewer or greater number could be provided so long as they achieve the spacing function.

A transverse dimension of the looped region is less than a transverse dimension of the mounting (anchoring) region. Stated another way, in the expanded configuration, the distance from the apex of the loop 40 to the central longitudinal axis is lees than the distance from the vessel engaging hook 72a or 72b to the central longitudinal axis.

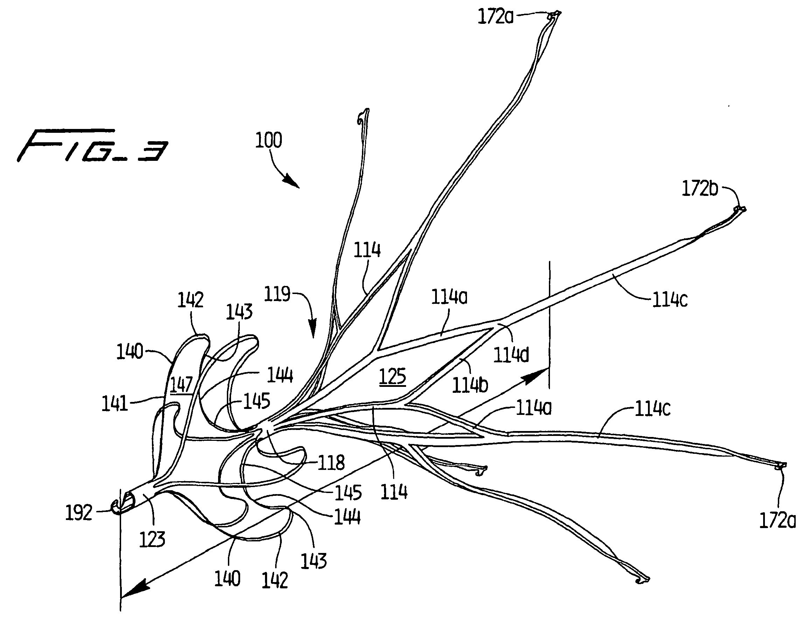

In the embodiment of the vein filter according to the present invention shown in Figure 3, filter 100 is identical to filter 10 except for the radially extending spacers 140 (The corresponding parts are labeled in the "100" series). The spacers 140 are in the form of closed loops. Preferably six loops are provided, positioned about 60 degrees apart, although other spacing and other number of loops could be provided. The spacer loops 140 are positioned distal of the filter portion 119 and proximal of the tubular portion 123 and retrieval hook 192 with respect to the direction of blood flow. Preferably they are formed integral with the filter.

The loops 140 are wing shaped such that a distal region 141 extends outwardly and radially in a direction toward the mounting portion (or caudal end) of the filter, reaches an apex region 142 and then bows inwardly at region 143 to extend at region 144 toward the retrieval end (or cranial end) of the filter, forming an inwardly bowed section, and then extends proximally at region 145 into the tubular portion 118. Thus, as shown, the loops 140 point in a direction toward the retrieval end of the filter.

To enable movement between an expanded and collapsed configuration, the filter of the embodiments described herein, as noted above, is preferably made of shape memory metal material, such as Nitinol, a nickel titanium alloy, and preferably manufactured from a laser cut tube. To facilitate passage of the filter through the lumen of the delivery sheath and into the vessel, cold saline is injected into the delivery sheath or catheter and around the filter in its collapsed position within the delivery sheath. This shape memory material characteristically exhibits rigidity in the austenitic state and more flexibility in the martensitic state. The cold saline maintains the temperature dependent filter in a relatively softer condition as it is in the martensitic state within the sheath. This facilitates the exit of the filter from the sheath as frictional contact between the filter and the inner surface of the sheath would otherwise occur if the filter was maintained in a rigid, i.e. austenitic, condition.

Once ejected from the delivery sheath or catheter, the filter is no longer cooled and is exposed to the warmer body temperature, which causes the filter to return towards its austenitic memorized configuration.

In the placement (expanded) configuration, the filter moves towards its memorized position and the extent it returns to its fully memorized position will be dependent on the size of the vessel in which the filter is inserted. (The larger the vessel, the closer the filter comes to returning to its fully memorized position). The extent of movement of the spacer(s) to its fully memorized position could also be limited by the size of the vessel.

The filter can be inserted through the jugular vein in the neck of the patient or through the femoral vein in the leg of the patient or the arm. The filters can also be placed in the superior vena cava.

Figures 13-15 of the '929 application illustrate delivery and placement of the filter 10, by way of example, in the inferior vena cava. The filters 10 and 100 disclosed herein can be inserted in the same manner. The delivery catheter is withdrawn to enable filter 10 to be warmed by body temperature to transition to the expanded placement configuration. The other filters described herein could be inserted in the same manner. Note it is implanted in the orientation such that filter section 19 is downstream of the flared section 17. This enables blood clots or other particles to be directed to the center of the filter section by the angled struts. Thus the direction of insertion, e.g. upstream or downstream direction, will determine how the filter is to be positioned in the delivery catheter. Also note in its implanted orientation, spacers 40 (or 140) are downstream of the filter section 19.

The foregoing filters can be removed from access through the internal jugular or femoral vein. Various methods can be used to remove the filter such as those described in commonly assigned co-pending application serial no. 11/801,547, filed May 10, 2007, (US-A-2010063535) and in the '429 application. Methods include for example, slotted hooks, graspers, etc.

A recess or cutout is preferably provided at the tubular end portion to form a hook portion 90 (Figure 1) and 190 (Figure 2). The curved hook 92 or 192 at the proximalmost end receives a snare or other device for removal and is described in detail in the '429 application. The hook 92 as shown in laterally offset from a central longitudinal axis of the filter. This facilitates passages of a guidewire through the filter.

When the filter is grasped by the retrieval device and pulled distally to disengage from the vessel walls, the spacers flex inwardly to collapse within the retrieval sheath or catheter for removal. When the filter is pulled into the retrieval sheath it is collapsed for removal.

To facilitate removal of the filter from the vessel, cold saline can be injected onto the implanted filter to change the temperature of the filter to move it to a relatively softer condition to facilitate the filter being drawn into the retrieval sheath. That is, injection of cold saline will cause the filter to approach its martensitic state, bringing the filter to a more flexible condition. The flexible condition facilitates the collapse and withdrawal of the filter into the retrieval sheath by decreasing the frictional contact between the filter and the inner surface of the retrieval sheath.

A delivery system which can be used for the filter of the present invention which includes a filter cartridge, is shown and described in the '429 application.

While the above description contains many specifics, those specifics should not be construed as limitations on the scope of the disclosure, but merely as exemplifications of preferred embodiments thereof. For example, the foregoing filters can be inserted in other regions of the body. Also, the foregoing filters can be made of materials other than shape memory material. Those skilled in the art will envision many other possible variations that are within the scope of the claims appended hereto.

## Claims

1. A vessel filter comprising a first region (119), a second region and a third region (140), the filter movable between a collapsed position for delivery to the vessel and an expanded position for placement within the vessel, the first region having a filter portion (119) having a converging region to direct particles toward the center of the filter, the first region including a plurality of spaced apart elongated struts (114), and a plurality of connecting struts (114a, 114b) extending at an angle from the elongated struts to form closed geometric shapes, the second region being flared in the expanded position having a transverse dimension increasing toward a second end portion opposite the filter portion, the second end portion being at the proximal region of the filter, the second region including vessel engaging hooks (172a, 172b) at the second end portion, the third region having a plurality of spacer struts (140) extending radially with respect to a longitudinal axis of the filter in the expanded position, **characterized in that** the spacer struts (140) form a plurality of integral closed wing-shaped looped struts positioned distally of the filter portion (119), each loop extending a distance from a central longitudinal axis (L) of the filter that is less than a distance the vessel engaging hook (172a, 172b) is from the central longitudinal axis.

2. A vessel filter as claimed in claim 1, wherein a transverse dimension of the filter at the region of the looped spacer struts (140) is less than a transverse dimension of the filter at the region of the vessel engaging portions (172a, 172b).

3. A vessel filter as claimed in claim 1 or 2, wherein the spacer struts (140) are substantially aligned with the longitudinal axis (L) of the filter in their collapsed position.

4. A vessel filter as claimed in claim 3, wherein in the expanded configuration an opening (143) in the loops (140) is transverse to a longitudinal axis (L) of the filter.

5. A vessel filter as claimed in any preceding claim, wherein the filter is formed from a laser cut tube and composed of shape memory material and the spacer struts (140) are formed integrally with the filter.

6. A vessel filter as claimed in any preceding claim, wherein the converging region terminates in a tubular portion (118) and each of the elongated struts (114) in the first region (119) extends outwardly from the tubular portion, and the spacer struts (140) extend radially from the tubular portion and are positioned distally of the elongated struts.

7. A vessel filter as claimed in claim 6, wherein the spacer struts (140) have a looped shape memory position and during delivery have a collapsed position substantially flush with the tubular portion (118).

8. A vessel filter as claimed in any preceding claim, wherein the spacer struts (140) extend radially from a first end of the filter in a first direction toward the proximal region of the filter to an end region and then curve inwardly in a second opposite direction toward the distal end of the filter.

9. A vessel filter as claimed in any preceding claim, wherein the filter includes a first tubular portion (118) proximal of the spacer struts (140) and a second tubular portion (123) distal of the spacer struts, the first and second tubular portions dimensioned to receive a guidewire therethrough.

10. A vessel filter as claimed in any preceding claim, wherein the spacer struts (140) are self expanding upon exposure from a delivery sheath.

11. A vessel filter as claimed in any preceding claim, further comprising a retrieval hook (192) at a distal end of the filter, the hook laterally offset from a central longitudinal axis (L) of the filter to facilitate passage of a guidewire.

12. A vessel filter as claimed in any preceding claim, wherein the filter comprises a body made from a single tube, the tube cut to create the plurality of elongated struts (114) and spacer struts (140) formed integrally with the filter.

13. A vessel filter as claimed in any preceding claim, wherein a wall of the filter separates the looped struts (140) from the filter portion (119).

## Patentansprüche

1. Gefäßfilter, der einen ersten Bereich (119), einen zweiten Bereich und einen dritten Bereich (140) umfasst, wobei der Filter zwischen einer zusammengeklappten Position für die Einführung in das Gefäß und einer ausgeklappten Position für die Platzierung in dem Gefäß beweglich ist, wobei der erste Bereich einen Filterabschnitt (119) mit einem zusammenlaufenden Bereich aufweist, um Teilchen zur Mitte des Filters zu leiten, wobei der erste Bereich eine Vielzahl von voneinander beabstandeten, länglichen Streben (114) und eine Vielzahl von verbindenden Streben (114a, 114b) einschließt, die sich in einem Winkel von den länglichen Streben erstrecken, um geschlossene geometrische Formen zu bilden, wobei der zweite Bereich in der ausgeklappten Position aufgeweitet ist, die eine Querabmessung aufweist, die in Richtung des zweiten Endabschnitts gegenüber dem Filterabschnitt zunimmt, wobei der zweite Endabschnitt an einem proximalen Bereich des Filters liegt, wobei der zweite Bereich in das Gefäß eingreifende Haken (172a, 172b) an dem zweiten Endabschnitt einschließt, wobei der dritte Bereich eine Vielzahl von Abstandstreben (140) aufweist, die sich in Bezug auf eine Längsachse des Filters radial in der ausgeklappten Position erstrecken, **dadurch gekennzeichnet, dass** die Abstandsstreben (140) eine Vielzahl von ganzheitlichen geschlossenen, flügelförmigen, gewundenen Streben bilden, die distal des Filterabschnitts (119) positioniert sind, wobei sich jede Windung in einem Abstand von einer mittigen Längsachse (L) des Filters erstreckt, der kleiner ist als ein Abstand des in das Gefäß eingreifenden Hakens (172a, 172b) von der mittigen Längsachse ist.

2. Gefäßfilter nach Anspruch 1, worin eine Querabmessung des Filters am Bereich der gewundenen Abstandsstreben (140) kleiner ist als eine Querabmessung des Filters am Bereich der in das Gefäß eingreifenden Abschnitte (172a, 172b).

3. Gefäßfilter nach Anspruch 1 oder 2, worin die Abstandsstreben (140) in ihrer zusammengeklappten Position im Wesentlichen mit der Längsachse (L) des Filters ausgerichtet sind.

4. Gefäßfilter nach Anspruch 3, worin in der ausgeklappten Konfiguration eine Öffnung (143) in den Windungen (140) quer zu einer Längsachse (L) des Filters vorliegt.

5. Gefäßfilter nach einem vorstehenden Anspruch, worin der Filter aus einem Laser-geschnittenen Röhrchen gebildet ist und aus einem Formgedächtnisstoff besteht und die Abstandsstreben (140) ganzheitlich mit dem Filter gebildet sind.

6. Gefäßfilter nach einem vorstehenden Anspruch, worin der zusammenlaufende Bereich in einem röhrenförmigen Abschnitt (118) endet und sich jede der länglichen Streben (114) in dem ersten Bereich (119) von dem röhrenförmigen Bereich nach außen erstreckt, und sich die Abstandsstreben (140) von dem röhrenförmigen Bereich radial erstrecken und distal der länglichen Streben positioniert sind.

7. Gefäßfilter nach Anspruch 6, worin die Abstandsstreben (140) eine gewundene Formgedächtnis-Position aufweisen und während der Einführung eine zusammengeklappte Position aufweisen, die im Wesentlichen bündig mit dem röhrenförmigen Abschnitt (118) ist.

8. Gefäßfilter nach einem vorstehenden Anspruch, worin sich die Abstandsstreben (140) von einem ersten Ende des Filters radial in eine erste Richtung zum proximalen Bereich des Filters zu einem Endbereich erstrecken und dann in einer zweiten entgegengesetzten Richtung zum distalen Ende des Filters nach innen gebogen sind.

9. Gefäßfilter nach einem vorstehenden Anspruch, worin der Filter einen ersten röhrenförmigen Abschnitt (118) proximal der Abstandsstreben (140) und einen zweiten röhrenförmigen Abschnitt (123) distal der Abstandsstreben einschließt, wobei der erste und zweite röhrenförmige Abschnitt zur Aufnahme eines Führungsdrahts dahindurch dimensioniert sind.

10. Gefäßfilter nach einem vorstehenden Anspruch, worin sich die Abstandsstreben (140) bei Freilegung aus einer Einführungshülle selbst ausklappen.

11. Gefäßfilter nach einem vorstehenden Anspruch, der weiter einen Rückholhaken (192) an einem distalen Ende des Filters umfasst, wobei der Haken von einer mittigen Längsachse (L) des Filters zur Begünstigung des Durchgangs eines Führungsdrahts seitlich versetzt ist.

12. Gefäßfilter nach einem vorstehenden Anspruch, worin der Filter einen Körper umfasst, der aus einem einzigen Röhrchen hergestellt ist, wobei das Röhrchen zur Entstehung der Vielzahl von länglichen Streben (114) und Abstandsstreben (140), die ganzheitlich mit dem Filter gebildet sind, geschnitten ist.

13. Gefäßfilter nach einem vorstehenden Anspruch, worin eine Wand des Filters die gewundenen Streben (140) von dem Filterabschnitt (119) trennt.

## Revendications

1. Filtre de vaisseau comprenant une première région (119), une deuxième région et une troisième région (140), le filtre étant mobile entre une position repliée pour la livraison au vaisseau et une position dilatée pour le placement au sein du vaisseau, la première région ayant une portion de filtre (119) ayant une région convergente pour diriger des particules vers le centre du filtre, la première région incluant une pluralité de montants allongés espacés (114) et une pluralité de montants de connexion (114a, 114b) s'étendant à un angle depuis les montants allongés pour former des formes géométriques fermées, la deuxième région étant évasée dans la position dilatée ayant une dimension transversale augmentant vers une seconde portion d'extrémité opposée à la portion de filtre, la seconde portion d'extrémité étant au niveau de la région proximale du filtre, la deuxième région incluant des crochets d'engagement de vaisseau (172a, 172b) au niveau de la seconde portion d'extrémité, la troisième région ayant une pluralité de montants écarteurs (140) s'étendant radialement par rapport à un axe longitudinal du filtre dans la position dilatée, **caractérisé en ce que** les montants écarteurs (140) forment une pluralité de montants intégraux en boucle en forme d'aile fermée positionnés de manière distale à la portion de filtre (119), chaque boucle s'étendant à une distance d'un axe longitudinal central (L) du filtre qui est inférieure à une distance de laquelle le crochet d'engagement de vaisseau (172a, 172b) est de l'axe longitudinal central.

2. Filtre de vaisseau selon la revendication 1, dans lequel une dimension transversale du filtre au niveau de la région des montants écarteurs en boucle (140) est inférieure à une dimension transversale du filtre au niveau de la région des portions d'engagement de vaisseau (172a, 172b).

3. Filtre de vaisseau selon la revendication 1 ou 2, dans lequel les montants écarteurs (140) sont essentiellement alignés avec l'axe longitudinal (L) du filtre dans leur position repliée.

4. Filtre de vaisseau selon la revendication 3, dans lequel une ouverture (143) dans les boucles (140) est transversale à un axe longitudinal (L) du filtre dans la configuration dilatée.

5. Filtre de vaisseau selon l'une quelconque des revendications précédentes, dans lequel le filtre est formé d'un tube découpé au laser et composé de matériau à mémoire de forme, et les montants écarteurs (140) sont intégralement formés avec le filtre.

6. Filtre de vaisseau selon l'une quelconque des revendications précédentes, dans lequel la région convergente se termine en une portion tubulaire (118) et chacun des montants allongés (114) dans la première région (119) s'étend vers l'extérieur depuis la portion tubulaire, et les montants écarteurs (140) s'étendent radialement depuis la portion tubulaire et sont positionnés de manière distale aux montants allongés.

7. Filtre de vaisseau selon la revendication 6, dans lequel les montants écarteurs (140) ont une position de mémoire de forme en boucle et ont une position repliée essentiellement à fleur avec la portion tubulaire (118) au cours de la livraison.

8. Filtre de vaisseau selon l'une quelconque des revendications précédentes, dans lequel les montants écarteurs (140) s'étendent radialement d'une première extrémité du filtre dans une première direction vers la région proximale du filtre à une région d'extrémité, puis se courbent vers l'intérieur dans une seconde direction opposée vers l'extrémité distale du filtre.

9. Filtre de vaisseau selon l'une quelconque des revendications précédentes, dans lequel le filtre inclut une première portion tubulaire (118) proximale aux montants écarteurs (140) et une seconde portion tubulaire (123) distale aux montants écarteurs, les première et seconde portions tubulaires étant dimensionnées pour recevoir un fil guide à travers elles.

10. Filtre de vaisseau selon l'une quelconque des revendications précédentes, dans lequel les montants écarteurs (140) sont à dilatation automatique suite à l'exposition depuis une gaine de livraison.

11. Filtre de vaisseau selon l'une quelconque des revendications précédentes, comprenant en outre un crochet de récupération (192) à une extrémité distale du filtre, le crochet étant décalé latéralement d'un axe longitudinal central (L) du filtre pour faciliter le passage d'un fil guide.

12. Filtre de vaisseau selon l'une quelconque des revendications précédentes, dans lequel le filtre comprend un corps constitué d'un seul tube, le tube étant découpé pour créer la pluralité de montants allongés (114) et de montants écarteurs (140) intégralement formés avec le filtre.

13. Filtre de vaisseau selon l'une quelconque des revendications précédentes, dans lequel une paroi du filtre sépare les montants en boucle (140) de la portion de filtre (119).
